# EUROPEAN PATENT SPECIFICATION

(11) **EP 0 624 597 B1**
(45) Date of publication and mention of the grant of the patent: **10.02.1999**
(21) Application number: 94830208.8
(22) Date of filing: 02.05.1994
(51) Int. Cl.: C07K 14/18, G01N 33/576

(54) **HCV peptides and uses thereof**
HCV-Peptide und deren Verwendung
HCV-peptides et l'usage de ceux-ci

(30) Priority: 12.05.1993 IT RM930309
(43) Date of publication of application: 17.11.1994
(73) Proprietor: WABCO B.V., 7940 KB Meppel (NL)
(72) Inventor: Rosa, Carlo, Saluggia (IT); Osborne, Stephen John, Saluggia (IT); Griva, Silvia, Saluggia (IT); Garetto, Fulvio, Saluggia (IT); Bonelli, Fabrizio, Saluggia (IT)
(74) Representative: de Simone, Domenico

(56) References cited:
- EP-A- 0 468 527
- EP-A- 0 489 968
- WO-A-93/06488
- WO-A-93/18054

### SEQUENCE LISTING

### (1) GENERAL INFORMATION:

(i) APPLICANT:
   (A) NAME: Sorin Biomedica S.p.A.
   (B) STREET: Via Crescentino
   (C) CITY: Saluggia
   (D) STATE: VC
   (E) COUNTRY: Italy
   (F) POSTAL CODE (ZIP): 13040
(ii) TITLE OF INVENTION: HCV peptides and uses thereof
(iii) NUMBER OF SEQUENCES: 3
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)

### (2) INFORMATION FOR SEQ ID NO: 1:

(i) SEQUENCE CHARACTERISTICS:
   (A) LENGTH: 17 amino acids
   (B) TYPE: amino acid
   (D) TOPOLOGY: linear
(ii) MOLECULE TYPE: peptide
(iii) HYPOTHETICAL: NO
(xi) SEQUENCE DESCRIPTION: SEQ ID NO: 1:
   Asn His Val Ser Pro Thr His Tyr Val Pro Glu Ser Asp Ala Ala Ala
   1               5                   10                  15
   Arg

### (2) INFORMATION FOR SEQ ID NO: 2:

(i) SEQUENCE CHARACTERISTICS:
   (A) LENGTH: 15 amino acids
   (B) TYPE: amino acid
   (D) TOPOLOGY: linear
(ii) MOLECULE TYPE: peptide
(iii) HYPOTHETICAL: NO
(xi) SEQUENCE DESCRIPTION: SEQ ID NO: 2:
   Glu Thr Trp Lys Lys Pro Asp Tyr Glu Pro Pro Val Val His Gly
   1               5                   10                  15

### (2) INFORMATION FOR SEQ ID NO: 3:

(i) SEQUENCE CHARACTERISTICS:
   (A) LENGTH: 34 amino acids
   (B) TYPE: amino acid
   (D) TOPOLOGY: linear
(ii) MOLECULE TYPE: peptide
(iii) HYPOTHETICAL: NO
(xi) SEQUENCE DESCRIPTION: SEQ ID NO: 3:
   Asn His Val Ser Pro Thr His Tyr Val Pro Glu Ser Asp Ala Ala Ala
   1               5                   10                  15
   Arg Gly Gly Glu Thr Trp Lys Lys Pro Asp Tyr Glu Pro Pro Val Val
               20                  25                  30
   His Gly

## Description

The invention relates to HCV peptides and to HCV derived chimeric peptides, comprising the dominant epitopes of non-structural proteins coded by NS4b and NS5a HCV genes, a process to obtain such peptides and uses thereof in developing immunodiagnostic kits.

HCV is known as being the main agent of non-A non-B hepatitis (1). The HCV genome consists of a RNA positive helix of 9,400 nucleotides appr., which codifies three structural proteins, namely: the core (C), two envelopes (env1 and env2) and six non-structural proteins (NS), i.e. NS2, NS3, NS4a, NS4b, NS5a and NS5b. Such RNA regions comprise non coding fragments both at 5'- and at 3'- terminus, thus showing a similar organisation as detected in Flavivirus and Pestivirus (2).

In order to prepare diagnostic kits to detect HCV infections, virus coding sequences both of structural and of non structural regions were expressed in E. coli, yeast and Baculovirus (3). The resulting proteins were used both as single protein or as combination thereof, to develop immunological kits that are able to capture anti-HCV anti antibodies from samples.

Data known in the art from these assays show that HCV is the main responsible agent for the post-transfusional sporadic hepatitis, and that the use of said proteins is very advantageous in analyzing blood samples from donors (6,7). However, with assays known in the art and available on the market, some samples escape from the screening and give false negative results.

Serological studies either on chimpanzees or on post-transfusion HCV infected patients showed that the most important epitopes reside into the nucleocapside protein, consisting of NS3 and NS4b products (8,9,11,12).

Recently it was reported (13) that also the gene for the NS5a product codifies a product able to efficiently capture anti-HCV antibodies. The authors expressed a recombinant protein in E. coli named (C)-NS3, that is able to bind both to anti-C and to anti-NS3 antibodies (13). The chimeric protein is suitable to develop a sensitive assay to detect anti-HCV antibodies.

Following a number of studies of serum conversion, in order to make available a proper test for the presence of anti-HCV antibodies in the blood from donors, the diagnostic kit must also contain the NS4b gene product, preferably the antigenic determinants thereof. This is confirmed through assays based on a NS4b derived polypeptide, known as c100-3, which, however, did not show satisfactory specificity and sensitivity.

It was recently found (7) that 90% appr. of the HCV induced hepatitis were detected by carrying out a test, based on an ELISA assay, comprising the nucleocapsid, the NS3 product and the c100-3 polypeptide.

However it is advantageous to include also the NS5a gene product as a sensitive marker for the HCV induced infection in order to make available a more sensitive assay.

Accordingly, in order to develop a suitable immunodiagnostic kit to detect HCV induced infections, having highest specifity and sensitivity levels, an obvious need exists to have available various HCV proteins, or fractions thereof. In order to prepare kits comprising more specific combined ligands, a further need exists to identify antigenic determinants of said proteins.

Among the many theoretical sequences, the authors identified the main antigenic determinants of NS4b and NS5a, and analyzed the immunoreactivity thereof in a HCV detection test, based on the identification of such epitopes. The authors also synthesized chemically such epitopes into one chimeric peptide, comprising both the epitopes, and demonstrated how advantageously this peptide could be used to detect anti-HCV antibodies, as having a specifity and a sensitivity, which are comparable to those of peptides containing the epitopes separately; however being more easily and economically used to develop immunodiagnostic kits on a large scale.

Accordingly, it is an object of the invention a peptide or a combination thereof comprising at least the amino acid sequences of SEQ ID No. 1 and of SEQ ID No. 2. Preferably the peptide comprises the amino acid sequence of SEQ ID No. 3.

It is a further object of the invention a method to detect in vitro anti-HCV antibodies from biological fluids through an immunologic assay, comprising at least the following steps:
- to pick up a biologic fluid sample from a subject;
- to incubate said sample together with an excess of a peptide or of a combination thereof according to the invention;
- to remove the sample not bound to said peptide or combination thereof; and
- to put in evidence the amount of bound antibodies to said peptide or combination thereof by using a detection system.

Further object is a diagnostic kit to detect in vitro anti-HCV antibodies from biologic fluids, comprising at least:
- an inert solid carrier, to which a peptide or a combination thereof according to the invention;
- a container with a buffer to dilute the biologic fluid sample;
- a container with a washing buffer;
- a container with anti-human IgG antibodies that are conjugated with a detecting system;
- a container with a positive control sample; and
- a container with a negative control sample.

The invention will be now described with reference to some clarifying, but not limiting Examples as well as to the annexed figures, wherein:
- fig.1 is a bar diagram showing different reactivities of 14 peptides from NS4b against the BBI serum panel;
- fig.2 is a table summarizing the NS4b-13 peptide (SEQ ID No. 1) reactivity against the BBI serum panel;
- fig.3 is a map of the NS5a peptide sequences;
- fig.4 shows the reactivity of peptides of fig.3 when tested by an ELISA assay with no. 2 (a), no. 11 (b), no. 24 (c) samples of the BBI serum panel, and with a HCV negative serum (d);
- fig.5 is a table summarizing the NS5a-44 peptide (SEQ ID No. 2) reactivity against the BBI serum panel;
- fig.6 is a table summarizing the NS4b-NS5a chimeric peptide (SEQ ID No. 3) reactivity against the BBI serum panel;
- fig.7 is a neutralisation test of the serum sample n.3, previously treated with the NS4b-13 peptide before the test with the NS4b-NS5b chimeric peptide;
- fig.8 is a neutralisation test of the serum sample n.11, previously treated with the NS5a-44 peptide before the test with the NS4b-NS5a chimeric peptide.

Market available sera are supplied by Boston Biomedica, Inc. (Massachussets, U.S.) (BBI serum panel). Donor blood samples are supplied by "Ospedale Santo Spirito" at Casale Monferrato, Italy. All the serum samples are maintained at - 20°C until the tests were carried out.

### EXAMPLE 1 - MAPPING OF EPITOPES OF NS4b AND NS5a GENE PRODUCTS AND CHIMERIC PEPTIDE SYNTHESIS

The NS4b and NS5a proteic sequences were analyzed using combined algorithms to predict potentially useful epitopes as capturing antigens. Depending upon hydrophilic and hydrophobic profiles of the HCV polyprotein sequence (18,19) as well as the predicted secondary structure thereof (20), 15 peptides of various sizes from NS4b sequence and 52 octapeptides from NS5a sequence are synthesized.

### IMMUNOASSAYS: PROCEDURES

The specificity of synthetic peptides is tested using 300 serum samples from blood donors, as well as through a further neutralisation assay, using separately both NS4b and NS5a epitopes as soluble antigens. The neutralisation assay is carried out as follows: samples no.3 and no.11 of the BBI serum panel, which react respectively only against C-100-3 and C 22C and against 5-1-1, C 33C, C 22C, according to the Ortho Riba II assay, are incubated with the NS4b and NS5a epitopes, or with a non correlated epitope, before being transferred to tray wells, coated with the peptide to be analyzed; sample amounts (20 µl) are mixed with PBS buffer (20 µl), containing decreasing amounts (from 5 µg up to 0.05 ng) of synthesized peptides.

After having been incubated for 18 hours at room temperature, mixtures are transferred on ELISA assay trays and the procedure, as described hereinafter, is carried out. Results are reported as binding activity percentages of peptides as compared to immunoreactivity when no soluble antigens are used.

### ANTI-HCV ELISA ASSAY

The antigenic activity is tested through indirect, solid phase ELISA assays, consisting of microtitration trays, that are prepared as follows: 1) synthetic peptides are solubilized with the smallest volume of 100% trifluoroacetic acid and then diluted to a final concentration of 5 µg/ml in a 0.05 M carbonate buffer, pH 9.6. Cysteine containing peptides, which must be maintained as linear monomers, are resuspended in citrate buffer, pH 4, to be used to coat the tray wells. The synthetic peptide is dissolved in 100% trifluoroacetic acid to 2 mg/ml. Then, the solution is diluted to 5 µg/ml with carbonate buffer, pH 9.6. 200 µl are added to each well and an incubation for 1 hour at 37°C is carried out; 2) wells are then washed out and blocked using a solution containing proteins (100 mM Tris-HCl, 0.2% BSA); 3) after having removed the fluid, wells are dried for 18 hours at room temperature and then sealed into polyethylene bags and maintained at 4°C until its use.

The assay is carried out as follows: peptide coated wells are incubated for 30 minutes at 37°C with 200 µl of 1:21 diluted serum, into the same solid phase blocking solution; then wells are washed 5 times with a 10 mM PBS, pH 7.4 buffer with 0.25% Triton x-100 . The enzyme tracer, consisting of 100 µl of goat anti-human IgG serum, is then added to each well after having been diluted with the diluent as above. After having been incubated for 30 minutes at 37°C, during which the enzyme tracer reacts with solid phase bound proteins, wells are washed with washing buffer; then, 100 µl of a solution containing a chromogen substrate (0.01% tetramethylbenzidine in 0.005% of H₂O₂) is added; after having been incubated for 30 minutes at room temperature, the reaction is blocked with 100 µl of a 2N H₂SO₄ blocking solution. Differential optical densities (at 450 nm and 630 nm) of the solution in each well are measured on a colorimeter (BIOTEK EL 311) and related to the presence of the antibody, which reacts with the peptide in solid phase.

Oligopeptides, which are synthesized directly in solid phase as previously described, are blocked using the same buffer, as described in the conventional ELISA assay.

### RESULTS

### MAPPING OF NS4b EPITOPES WITH SYNTHETIC PEPTIDES NS4b PEPTIDE SYNTHESIS

Peptide synthesis is carried out using an automatic Milligen 9050 synthesizer (Millipore Co., Ma, U.S.) using a Fmoc chemical technique. Peptide purity levels are checked through reverse phase HPLC and result in the range 85-90%. Amino acid sequences are checked by a PI2090 E microsequencer. The amino acid numbered positions refer to those used in the PCT Application No. W092/03458. Sequences of the 14 synthesized peptides are shown in Table 1 herebelow.

**Table 1**

| NS4b PEPTIDES | |
|---|---|
| Peptide | Sequence |
| NS4b-1 | 1688-1720 |
| NS4b-2 | 1710-1721 |
| NS4b-3 | 1725-1734 |
| NS4b-4 | 1734-1753 |
| NS4b-5 | 1742-1758 |
| NS4b-6 | 1781-1796 |
| NS4b-7 | 1794-1805 |
| NS4b-8 | 1861-1881 |
| NS4b-9 | 1867-1881 |
| NS4b-10 | 1902-1918 |
| NS4b-11 | 1911-1926 |
| NS4b-12 | 1918-1935 |
| NS4b-15 | 1924-1935 |
| NS4b-13 | 1926-1943 |
| NS4b-14 | 1935-1952 |

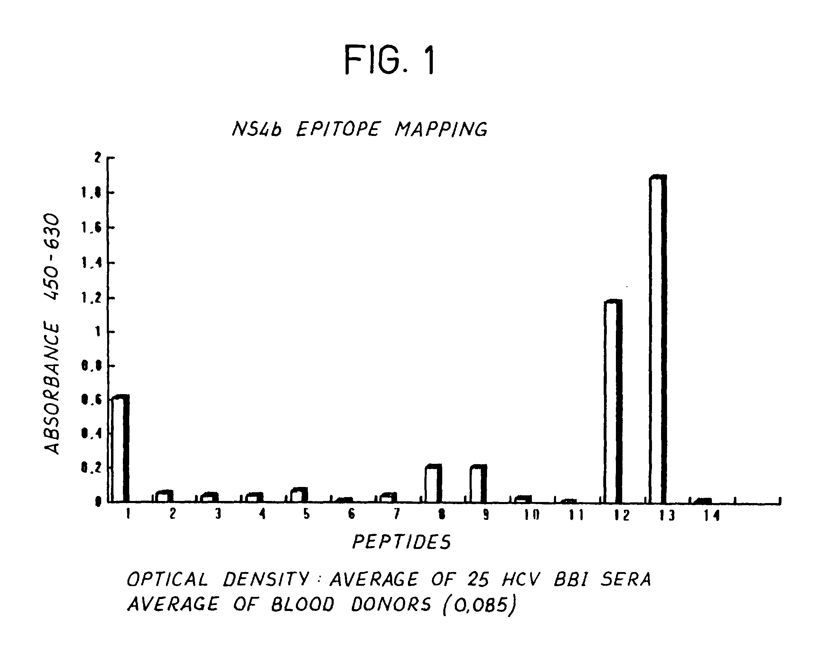

The results of an assay on 25 samples, which are positive in respect of anti-HCV antibodies of BBI serum panel, with the 15 synthesized peptides from NS4b are shown in fig.1. Numbers of amino acid positions refer to those shown in the PCT No. WO92/03458.

Values are calculated as cumulated absorbance (i.e. 450 nm optical density less 630 nm optical density, divided by the number of the samples, less the optical density average from the donor blood samples). Major antigenic domains map in the following peptides: NS4b-1 (1688-1720), NS4b-12 (1918-1935) and NS4b-13 (1926-1943). However the 1688-1720 peptide, when tested with some sample from blood donors, show a too high percentage (4%) of false positive reactions, while the 1918-1935 and 1926-1943 peptides show a high specificity level.

In order to better define the minimum epitope into the two peptides, the NS4b-15 (1924-1935) is analyzed. This peptide reacts more than the 1918-1935 peptide, but less than the 1926-1943 peptide. Accordingly, the 1926-1943 region is critical in order to define the highest sensitivity.

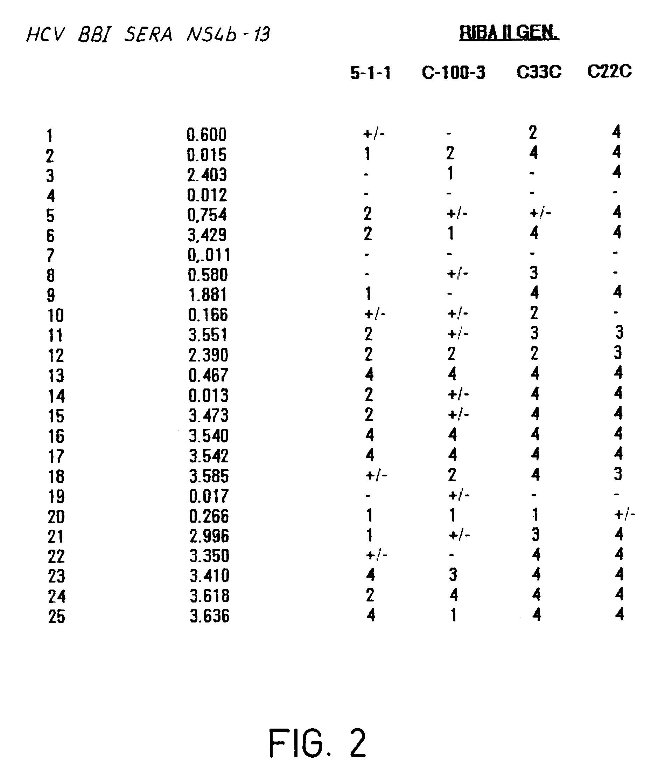

As shown in fig.2, the NS4b-13 peptide is used in an ELISA assay with different sera of the BBI panel and found to be immunoreactive for 19 samples out of 25. NS4b sequence is shown in SEQ ID No. 1.

### NS5a EPITOPE MAPPING WITH SUPERIMPOSING OCTAPEPTIDES NS5a SYNTHESIS

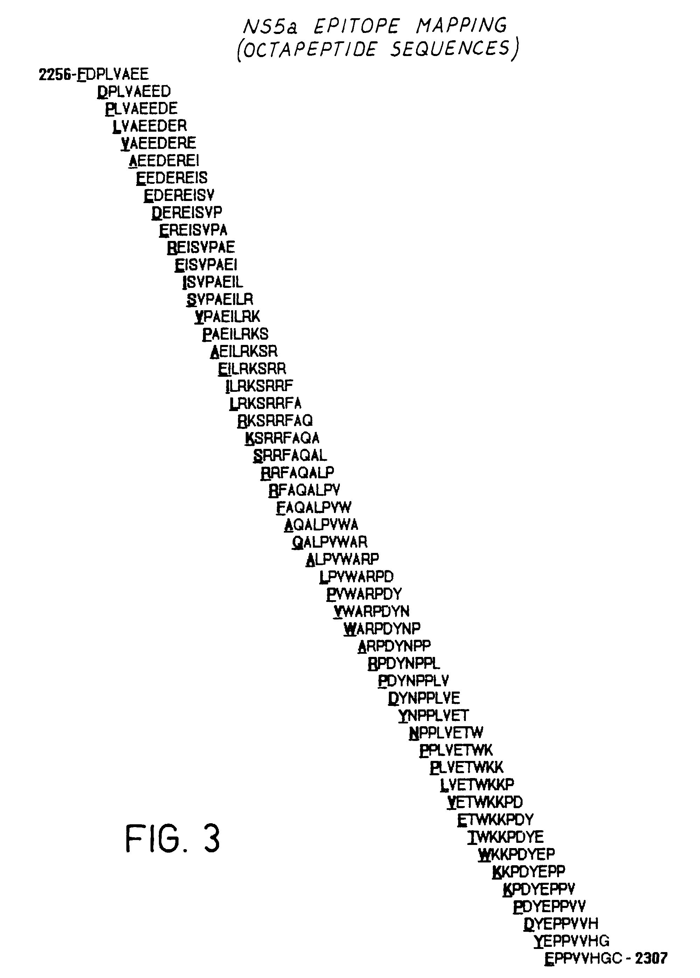

Octapeptides superimposing for only one amino acid, comprised into the highest hydrophilic region of the NS5a gene product, are synthesized on polyethylene substrates using materials supplied by Cambridge Research Biochemicals, Inc., Cambridge, U.K., according to the Manufacturer's instructions (Epitope Scanning Pin Kit). The process is carried out by combining activated esters for Fmoc with an HOBT (t-butyloxycarboxyl) for protection purposes. This method is described by Geysen and al. (14, 16) and is carried out as modified, as specified in (21). In such a way 52 octapeptides are synthesized throughout the more hydrophobic domain of the NS5a polypeptide, having the sequences as shown in fig.3.

Preliminary ELISA assays show that there is a no specific binding among human IgG from donor blood and substrates bearing the octapeptides, when a serum dilution less than 1:1000 are used, thus allowing to define the useful serum dilution to get a positive sample.

The substrate immunoreactivity is evaluated using three samples of the BBI serum panel (namely nos. 2, 11 and 24 samples), already previously classified as having an high anti-NS5a titer, according to the commercially available Inno-Lia assay (Innogenetics, Belgium).

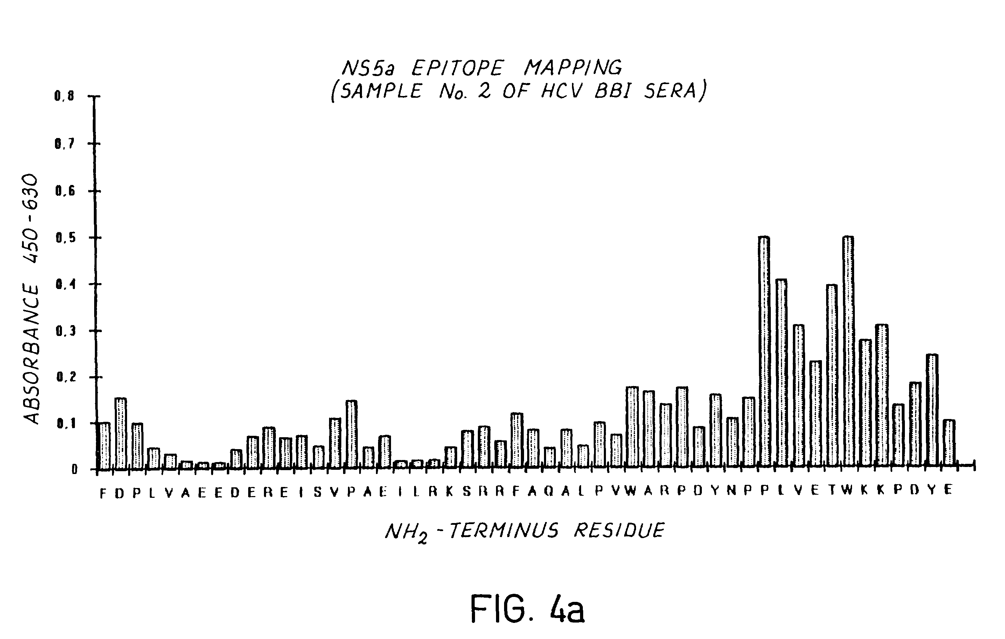

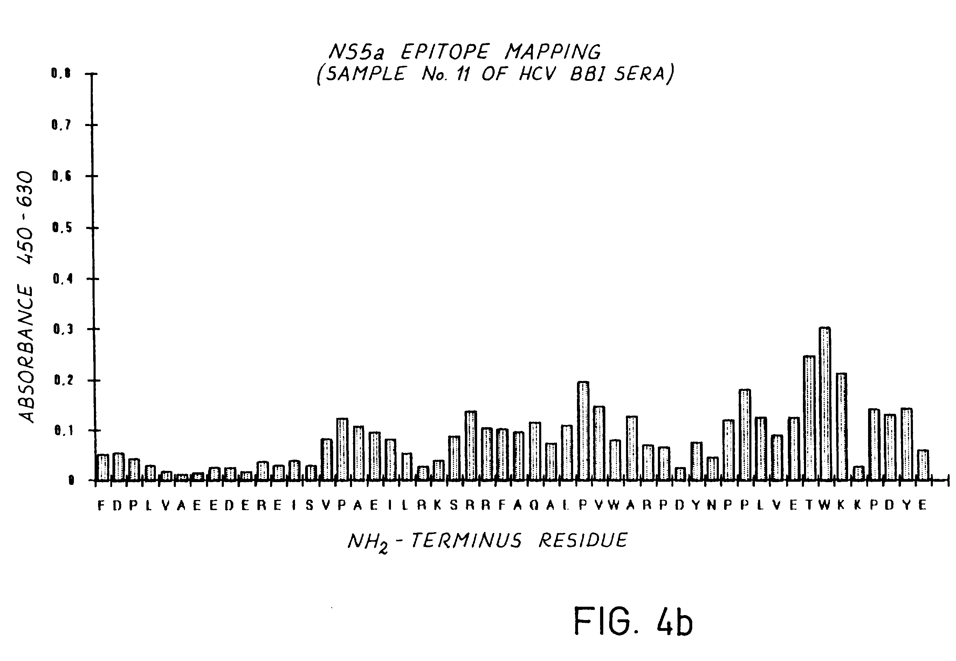

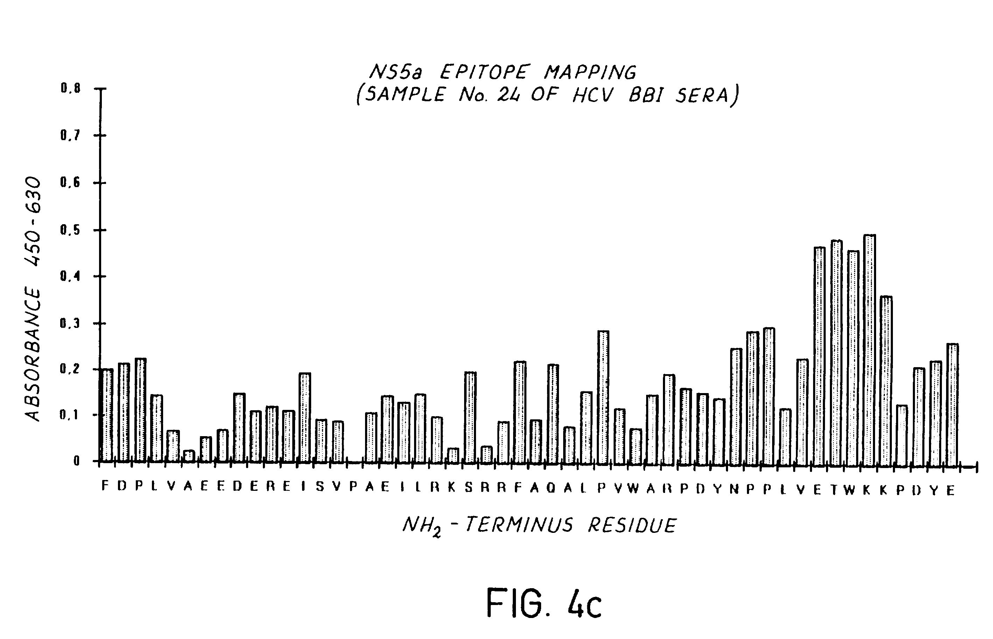

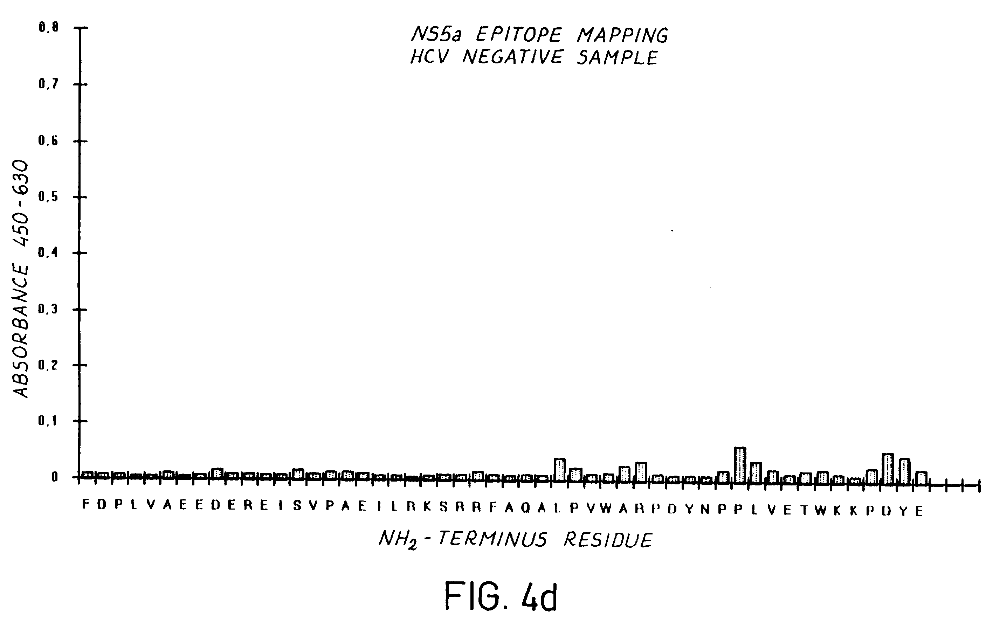

The ELISA assay results, based on the octapeptides with the three samples of sera of the BBI panel and the donor's blood sample, are shown in the fig. 4a, 4b, 4c and 4d. Data show a significant reactivity for most of the analyzed samples; anywhere the most reactive region comprises the sequence from aa. 1 to aa. 8 of SEQ ID No. 2.

The results of the ELISA assays with the octapeptides are highly reproducible and control samples, incubated with positive and negative sera, show that this procedure is effective in fully removing the substrate bound IgGs.

After having the epitopes been defined, a peptide, having the SEQ ID No. 2 sequence (NS5a-44) is synthesized.

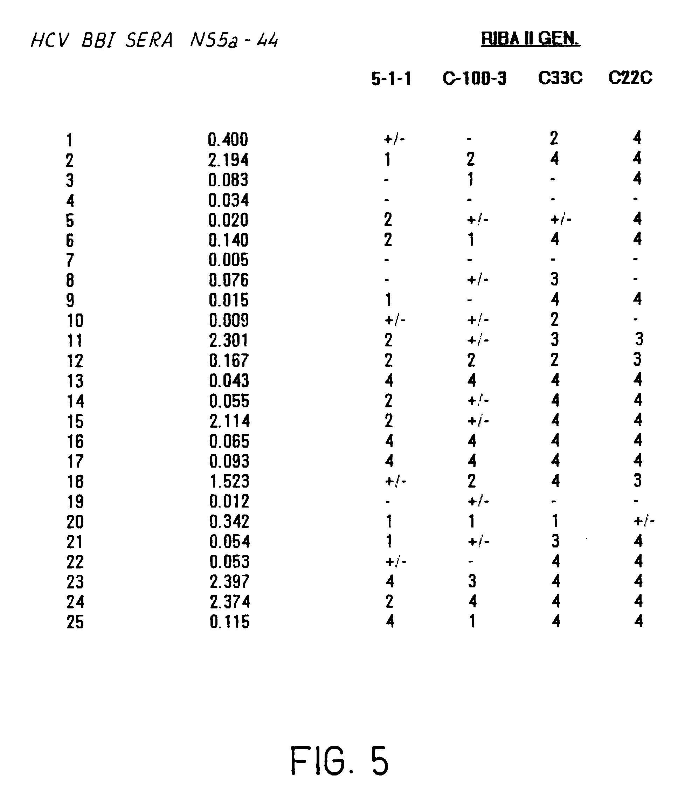

As shown in fig.5, this peptide is subjected to an ELISA assay, showing that the peptide is immunoreactive against the same three samples that were used in the epitope mapping; moreover, this peptide shows the ability to detect furthermore four other samples from the BBI serum panel.

This peptide does not show any false positive reaction when used with donor's blood samples.

### CHIMERIC PEPTIDE SYNTHESIS

After having been defined the minimum immunodominant sequences for both NS4b and NS5a peptides, a chimeric peptide, comprising the NS4b and NS5a peptides, spaced by two glycine residues, is synthesized, having the sequence as follows (SEQ ID No. 3):

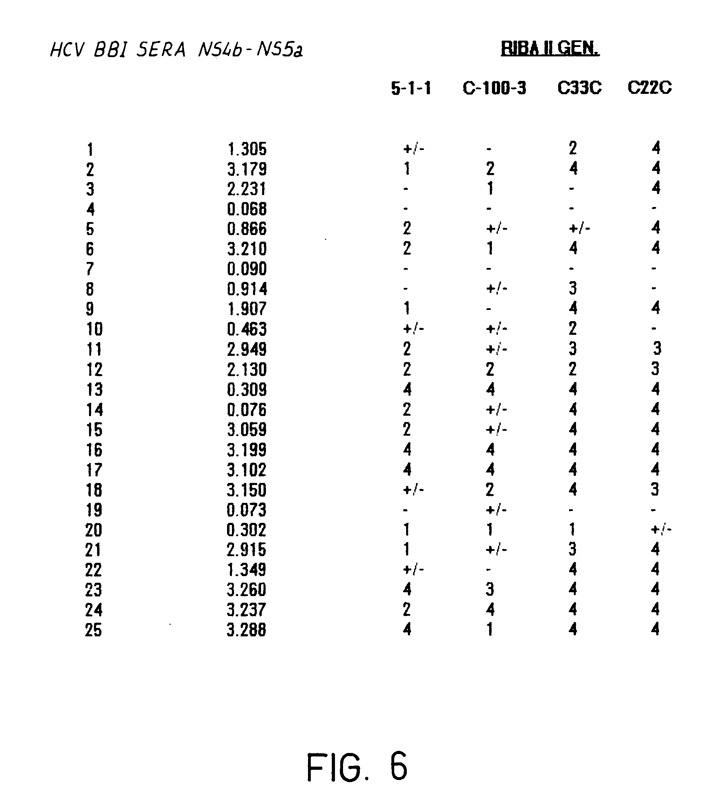

The chimeric peptide is analyzed through an indirect ELISA assay with the BBI serum panel, and the results are shown in fig.6. 21 sample of anti-HCV positive sera are captured by the immobilized chimeric peptide, with the ELISA assay.

Also the specificity in respect of 300 blood donor samples is tested and no false positive reaction detected (average value: 0.085; standard deviation: 0.028).

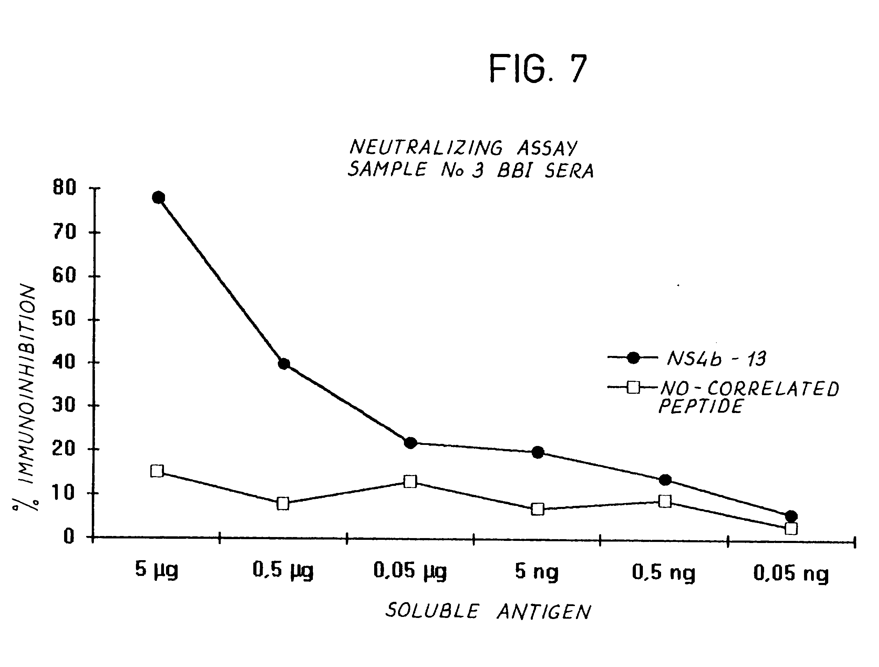

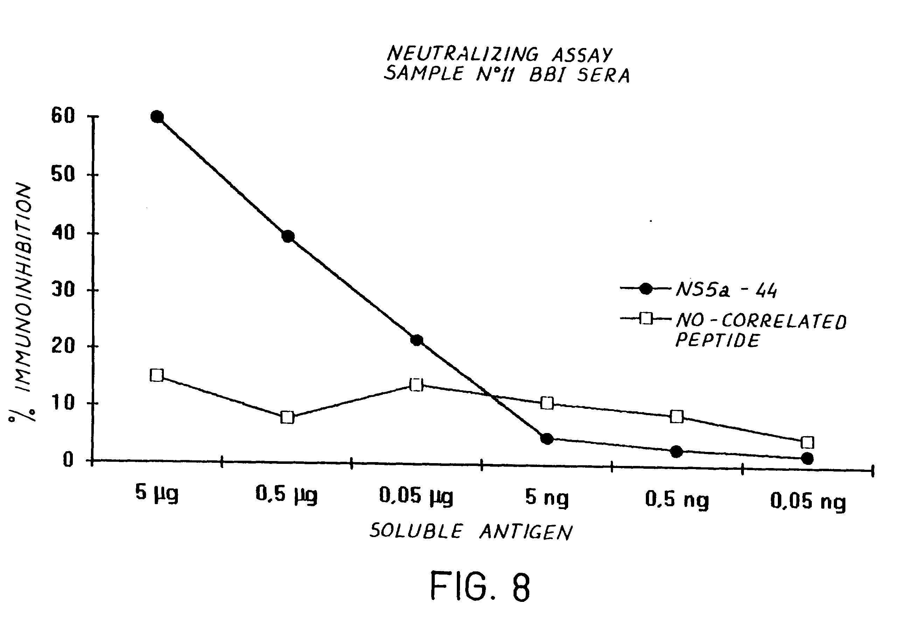

To carry out the neutralisation assay, no. 3 and 11 samples of the BBI serum panel are used. As shown in fig.7, a pre-incubation of the no.3 sample with the soluble NS4b-14 peptide before adding the NS4b-NS5a chimeric peptide to the coated wells, causes an immunoinhibition of the binding up to 80%; while no significant inhibition occurs when a non correlated peptide is used. In a similar way, the no. 11 sample binding shows to be inhibited up to 60%, after having been incubated with the NS5a-44 peptide, while no significant inhibition occurs when a non correlated peptide is used, as shown in fig.8.

The invention has been described with reference to specific peptides, but it us to be understood that the method and the diagnostic kit may comprise further HCV peptides not specifically disclosed in the instant specification but having reactivities comparable.

### REFERENCES

1. Choo, Q.L.and al.: Science 244, 359-362, 1989.
2. Grakoni, A. and al.: Journ. of Virology 67, 1385-1395, 1993.
3. Nasoff, M.S. and al.: Proc. Natl. Acad. Sci. USA 88, 5462-5466, 1991.
4. Kuo, G. and al.: Science 244, 362-364, 1989.
5. Chiba, J. and al.: Proc. Natl. Acad. Sci. 88, 4641-4645, 1991.
6. Alter, M.G. and al.: JAMA 264, 2231-2235, 1990.
7. Lelie, P.N. and al.: Journ. of Medical Virology, vol.37, 203-209.
8. Beach, M.J. and al.: Journ. of Medical Virology, 36, 226-237, 1992.
9. Vallari, D.S. and al.: Journ. Clin. Microbiol., 30, 552-556, 1992.
11. Desai, S.M. and al.: 1st annual meeting, July 6-9 1992,Fondazione Cini, Venice, Italy
12. Lesniewki R.R. and al.: 1st annual meeting, July 6-9, 1992, Fondazione Cini, Venice, Italy
13. Chien, D.Y. and al.: Proc. Natl. Acad. Sci. USA, 89, 10011-10015, 1992.
14.Osborne S.E. and al.: Proceedings of the 1993 Miami Biotechnology Winter Symposium; edited by Brew, K., Petsko G.A., Ahmad, F., Bialy, H., Black, S., Fernandez, A, Fenna, R.E., Lee, E.Y.C. and Whelan, W.J., pag.68.
15. Dawson, G.J. and al.: J. Clin. Microbiol., 29, 551-556, 1991.
16. Nicholson, S. and al.: J. of Virological Methods, 33, 311-317.
17. Alter, M.G. and al.: The New England Journal of Medicine, 27, 1899-1905, 1993.
18. Hopp, T.P. and Woods, K.R.: Proc. Natl. Acad. Sci. USA, 78, 3824, 1981.
19. Kyte, J. and Doolittle, R.F.: J. Mol. Biol., 157, 105, 1982.
20.Chou, P.Y. and Fasman, G.P., Biochemistry, 13, 22, 1974.
21. Cambridge Research Biochemicals Epitope scanning Pin recorder Kit.

## Claims

1. A peptide or a combination thereof with antigenic determinants of NS4b and NS5a of HCV comprising at least the amino acid sequences of SEQ ID No. 1 and of SEQ ID No. 2.

2. A peptide according to claim 2 comprising the amino acid sequence of SEQ ID No. 3.

3. A method to detect in vitro anti-HCV antibodies from biological fluids through an immunologic assay, comprising at least the following steps:
- to pick up a biologic fluid sample from a subject;
- to incubate said sample together with an excess of a peptide or of a combination thereof according to any of claims 1 or 2;
- to remove the sample not bound to said peptide or combination thereof; and
- to put in evidence the amount of bound antibodies to said peptide or combination thereof by using a detection system.

4. Diagnostic kit to detect in vitro anti-HCV antibodies from biologic fluids, comprising at least:
- an inert solid carrier, to which a peptide or a combination thereof according to any of claims 1 or 2 is bound;
- a container with a buffer to dilute the biologic fluid sample;
- a container with a washing buffer;
- a container with anti-human IgG antibodies that are conjugated with a detecting system;
- a container with a positive control sample; and
- a container with a negative control sample.

## Patentansprüche

1. Peptide oder dessen Kombination mit Antigen-Determinanten von NS 4b und NS 5a von HCV, enthaltend mindestens die Aminosäure-Sequenzen von SEQ ID No. 1 und von SEQ ID No. 2.

2. Peptide nach Anspruch 2 enthaltend die Aminosäure-Sequenze von SEQ ID No. 3.

3. Verfahren zur in-vitro Ermittlung von Anti-HCV-Antikörpern von biologischen Flüssigkeiten durch immunologische Proben, das mindestens die folgenden Verfahrenschritte enthält:
- Entnahme einer biologischen Flüssigkeit-Probe von einem menschlichen Wesen;
- Inkubation dieser Probe zusammen mit einem Überchuss von Peptide oder dessen Kombination nach einem der Ansprüche 1 oder 2;
- Entfernung der mit dem vorgenannten Peptide oder dessen Kombination nicht gebundenen Probe und
- Klarstellen der Menge von an dieses Peptide oder dessen Kombination gebundenen Antikörper durch Anwendung eines Ermittlungs systems.

4. Diagnostik-Kit zur Ermittlung von Anti-HCV-Antikörpern von biologischen Flüssigkeiten, enthaltend mindestens:
- einen inerten Festkörper, an den das Peptid oder dessen Kombination nach Anspruch 1 oder 2 gebunden ist;
- einen Behälter mit einem Puffer zur Verdünnung der biologischen Flüssigkeitsprobe;
- einen Behälter mit einem Waschpuffer;
- einen Behälter mit Antiglobulin-lgG-Antikörpern, die mit einem Ermittlungssystem konjugiert sind;
- einen Behälter mit einer positiven Prüfungsprobe;
- einen Behälter mit einer negativen Prüfungsprobe.

## Revendications

1. Peptide ou une combination du même avec des déterminants antigéniques de NS4b and NS5a contenant au moins les séquences des aminoacides de SEQ ID No. 1 et de SEQ ID No. 2.

2. Peptide selon la revendication 2, comprenant la sequence aminoacide de SEQ ID No. 3.

3. Procédé pour detecter in vitro de anticorps de anti-HCV des fluides biologiques par un essai immunologique, comprenant au moins les phases suivantes:
- prélèvement d'un échantillon de fluide biologique d'un sujet;
- incubation dudit échantillon avec un excès d'un peptide ou d'une combination du même selon une quelconque des revendications 1 ou 2;
- levée d'échantillon non lié audit peptide ou à la combination du même;
- mise en évidence de la quantité des anticorps liés audit peptide ou à la combination du même en utilisant un système de détection.

4. Kit diagnostique pour détection in vitro des anticorps anti HCV des fluides biologiques, comprenant au moins:
- un véhicule solide inerte, auquel est lié un peptide ou une combination du même selon une quelconque des revendication 1 ou 2;
- un récipient avec une solution tampon pour diluer l'échantillon du fluide biologique;
- un récipient avec une solution tampon de lavage;
- un récipient avec des anticorps de antiglobulin IgG, qui sont conjugués avec un système de détection;
- un récipient avec un système de contrôl positif;
- un récipient avec un système de contrôl negatif.
